# FASCICULE DE BREVET EUROPEEN

(11) **EP 3 414 337 B1**
(45) Date de publication et mention de la délivrance du brevet: **26.08.2020**
(21) Numéro de dépôt: 17703443.6
(22) Date de dépôt: 10.02.2017
(51) Int. Cl.: C12Q 1/04, C12Q 1/18, C12Q 1/22

(54) **COMPOSITION GELIFIEE DE SEL DE TETRAZOLIUM ET DE GOMME GELLANE**
GELARTIGE ZUSAMMENSETZUNG MIT TETRAZOLIUMSALZ UND GELLAN
GELLED COMPOSITION COMPRISING TETRAZOLIUM SALT AND GELLAN GUM

(30) Priorité: 11.02.2016 FR 1651100
(43) Date de publication de la demande: 19.12.2018
(73) Titulaire: Proteus, 30000 Nîmes (FR)
(72) Inventeur: PERSILLON, Cécile, 30000 Nimes (FR); ULLMANN, Christophe, 30000 Nimes (FR)
(74) Mandataire: Regimbeau
(86) Numéro de dépôt international: PCT/EP2017/052976
(87) Numéro de publication internationale: WO 2017/137543

(56) Documents cités:
- EP-A1- 2 562 169
- EP-A2- 0 322 591
- WO-A2-2008/094202
- US-A- 4 129 483
- MCLAUGHLIN M R ET AL: "Enhanced contrast of bacteriophage plaques in Salmonella with ferric ammonium citrate and sodium thiosulfate (FACST) and tetrazolium red (TZR)", JOURNAL OF MICROBIOLOGICAL METHODS, ELSEVIER, AMSTERDAM, NL, vol. 65, no. 2, 21 septembre 2005 (2005-09-21), - 1 mai 2006 (2006-05-01), pages 318-323, XP027926795, ISSN: 0167-7012 [extrait le 2006-05-01]

## Description

### Domaine technique

La mesure de la viabilité et de la prolifération cellulaire constitue la base de nombreux essais *in vitro* afin d'évaluer la réponse d'une population de cellules à des facteurs extérieurs. La réduction des sels de tétrazolium est un moyen fiable d'examiner la prolifération et/ou la viabilité cellulaire.

### Art Antérieur

Afin de contrôler efficacement la contamination d'une surface, il est fondamental de mesurer la viabilité cellulaire et/ou de vérifier la prolifération cellulaire. Plusieurs méthodes sont actuellement connues et peuvent se classifier comme des méthodes microbiologiques ou non microbiologiques.

Les méthodes microbiologiques sont des méthodes exigeant plusieurs jours d'attente avant l'obtention de résultats et demandant le plus souvent un savoir-faire particulier lié à la pratique de laboratoire ou à la stérilité. Pour répondre aux exigences actuelles de diminution des délais de production, il est nécessaire de réduire le temps d'obtention des résultats et cela est souvent possible grâce aux méthodes non microbiologiques qui comprennent des techniques très différentes.

La méthode d'ATPmétrie est une méthode de bioluminescence qui permet d'évaluer la quantité d'adénosine triphosphate en utilisant le couple enzyme/substrat luciférine/luciférase. Il est alors possible d'avoir une estimation rapide de la biomasse totale contenue dans un échantillon. Néanmoins, cette méthode nécessite un appareillage spécifique ainsi qu'un savoir-faire pour l'utiliser.

La PCR quantitative est une méthode donnant des résultats fiables et précis mais cette technique a plusieurs inconvénients. En effet, l'utilisation se fait en laboratoire avec un matériel spécifique et il est nécessaire d'avoir une certaine connaissance pour utiliser cette méthode et parfois même pour interpréter ses résultats. De plus, elle ne donne pas d'information sur la viabilité des microorganismes détectés mais seulement sur leur présence.

Ainsi, les techniques d'ATPmétrie et de PCR-quantitative se révèlent être des méthodes très difficiles à mettre en œuvre pour pouvoir tester directement en local, sur site industriel, des échantillons.

La détection par colorimétrie est une méthode de détection simple et efficace utilisant des réactifs connus qui détectent le plus souvent des protéines, des lipides ou des acides nucléiques comme par exemple les réactifs mis en jeu dans la méthode de Kjeldahl ou du biuret.

Cependant, la présence de protéines, de lipides ou d'acides nucléiques ne signifie pas forcément la présence de cellules et encore moins la présence de cellules vivantes. De ce fait, tous les réactifs colorimétriques ne permettent pas de détecter la viabilité cellulaire et il est nécessaire de choisir avec précision les types de réactifs à utiliser. Les réactifs les plus intéressants pour détecter la viabilité cellulaire et la prolifération semblent être les sels de tétrazolium.

Les protocoles utilisant des sels de tétrazolium sont le plus souvent des protocoles de laboratoire dont les résultats sont obtenus via une mesure par colorimétrie. Cela suppose donc que la composition comprenant le sel de tétrazolium soit liquide. Toutefois, un test de détection comprenant une composition liquide pose des problèmes pour un test d'échantillon en local.

En effet, l'obtention des résultats ainsi que l'observation de la coloration due aux sels de tétrazolium doit être rapide, il est donc nécessaire de faire une observation à l'œil nu. Le fait que l'échantillon puisse se diffuser dans la composition liquide va diminuer la visibilité du signal par dilution. L'utilisation d'une composition gélifiée devrait permettre de résoudre ce problème en concentrant le signal sur un point.

De plus, une composition gélifiée devrait permettre une meilleure robustesse du résultat car contrairement à la composition liquide, aucune dilution ne s'effectue entre l'échantillon et la composition gélifiée. Pour finir, un test mettant en jeu une composition gélifiée devrait donner un résultat avec une meilleure reproductibilité en éliminant différents facteurs inhérents à une composition liquide comme l'agitation volontaire ou involontaire.

De ce fait, la gélification d'une composition liquide comprenant un réactif colorimétrique devrait apporter de nombreux avantages notamment dans un kit de détection de viabilité faisant intervenir un ou des sels de tétrazolium.

Toutefois, les inventeurs ont constaté que la gélification de compositions contenant des sel(s) de tétrazolium posait des problèmes du fait que certains gélifiants modifient la couleur de base de la composition, donnent un gel cassant ou activent spontanément la coloration du ou des sels de tétrazolium.

Après de nombreuses recherches, les inventeurs ont trouvé le moyen de gélifier une composition comprenant un ou des sels de tétrazolium sans rendre inexploitables les résultats de la coloration. Pour cela les inventeurs, plutôt que d'utiliser des gélifiants standards, utilisent de la gomme gellane.

La gomme gellane est un polyoside extracellulaire issu *de Pseudomonas elodea* normalement utilisée en microbiologie dans les milieux de cultures pour sa thermostabilité dans les hautes températures. Il s'avère que ce composé permet une gélification de la composition qui ne dénature pas et n'active pas spontanément le ou les sels de tétrazolium tout en créant un gel non cassant dont la coloration permettra une bonne visualisation du signal à l'œil nu.

La demande de brevet US2010291539 décrit une composition pour la détection de bactériophages comprenant au moins un sel de tétrazolium, un tampon et de la gomme gellane. Cependant, cette composition est soit stérile et liquide (avant l'ajout de bactéries hôtes) soit non-stérile et gélifiée (après l'ajout des bactéries hôtes).

La demande de brevet US4129483 décrit une composition pour la détection de microorganismes viables comprenant au moins un sel de tétrazolium, un tampon et de l'agar mais ne décrit ni ne suggère d'utiliser de la gomme gellane en tant qu'agent gélifiant.

### Résumé

La présente invention concerne donc des compositions gélifiées à partir de gomme gellane comprenant au moins un composé de détection de viabilité cellulaire choisi parmi les sels de tétrazolium, leurs utilisations dans un kit de détection et des procédés mettant en œuvre lesdites compositions. Ces compositions sont avantageusement stériles.

### Mode de réalisation

L'invention a ainsi pour objet une composition gélifiée comprenant au moins un sel de tétrazolium, de la gomme gellane et un tampon. Ladite composition est avantageusement stérile. Ladite composition est destinée plus particulièrement à être utilisée pour la détection de la présence de microorganismes viables dans un échantillon.

Pour cela, ladite composition est effective sans ajout de source complémentaire d'énergie, de carbone ou d'azote. Sans vouloir être limité, ladite composition permet la détection de la présence de microorganismes viables à travers l'observation seule de leur respiration, indépendamment de leur croissance.

Selon l'invention, on entend par microorganismes tout organisme unicellulaire ou pluricellulaire invisible à l'œil nu, tel que des bactéries, des algues, des champignons ou des levures. De préférence, ceux-ci ne comprennent pas les bactériophages ou les virus.

Selon l'invention, on entend par sel de tétrazolium tout composé comprenant un anneau de tétrazolium pouvant être réduit. On peut citer à titre d'exemple comme sel de tétrazolium et sans vouloir se limiter le MTT (bromure de 3-(4,5-diméthylthiazolyl-2)-2,5-diphényltétrazolium), le TTC (chlorure de 2,3,5-triphényl-2H-tétrazolium), l'INT (2-(4-iodophényl)-3-(4-nitrophényl)-5-phényl-2H-tétrazolium), le MTS (5-[3-(carboxyméthoxy)phényl]-3-(4,5-diméthyl-2-thiazolyl)-2-(4-sulfophényl)-2H-tétrazolium), le WST-1 (5-(2,4-disulfophényl)-2-(4-iodophényl)-3-(4-nitrophényl)-2H-tetrazolium sodique), le WST-8 (2-(2-méthoxy-4-nitrophényl)-3-(4-nitrophényl)-5-(2,4-disulfophényl)-2H-tétrazolium sodique), le XTT (2,3-bis(2-méthoxy-4-nitro-5-sulfophényl)-5-[(phénylamino)-carbonyl]-2H-tétrazolium sodique) ou un mélange de ceux-ci, plus préférentiellement le MTT, le TTC, l'INT, le WST-8 ou un mélange de ceux-ci.

De préférence, la composition gélifiée, avantageusement stérile, telle que décrite précédemment est caractérisée en ce que au moins un sel de tétrazolium utilisé est du MTT, du TTC, de l'INT ou du WST-8.

Le bromure de 3-(4,5-diméthylthiazolyl-2)-2,5-diphényltétrazolium ou MTT est un sel de tétrazolium jaune pouvant être réduit notamment par l'action des enzymes déshydrogénases. Ainsi, dans les cellules métaboliquement actives les enzymes vont transformer le MTT jaune en formazan pourpre et générer des équivalents réducteurs tels que le NADH et le NADPH.

Le chlorure de 2,3,5-triphényl-2H-tétrazolium ou TTC est un sel de tétrazolium incolore pouvant être réduit notamment par l'action des enzymes déshydrogénases. Ainsi, dans les cellules métaboliquement actives les enzymes vont transformer le TTC incolore en 1,3,5-triphénylformazan pourpre et générer des équivalents réducteurs tels que le NADH et le NADPH.

Le chlorure de 2-(4-iodophényl)-3-(4-nitrophényl)-5-phényl-2H-tétrazolium ou INT est un sel de tétrazolium incolore pouvant être réduit notamment par l'action des enzymes déshydrogénases. Ainsi, dans les cellules métaboliquement actives les enzymes vont transformer l'INT incolore en formazan pourpre et générer des équivalents réducteurs tels que le NADH et le NADPH.

Le 2-(2-méthoxy-4-nitrophényl)-3-(4-nitrophényl)-5-(2,4-disulfophényl)-2H-tétrazolium sodique ou WST-8 est un sel de tétrazolium légèrement jaune pouvant être réduit notamment par l'action des enzymes déshydrogénases. Ainsi, dans les cellules métaboliquement actives les enzymes vont transformer le WST-8 légèrement jaune en formazan orange et générer des équivalents réducteurs tels que le NADH et le NADPH.

Le tampon permettra plus particulièrement de maintenir le pH de la composition gélifiée dans la gamme d'environ 6,5 à environ 7,5, plus particulièrement à un pH d'environ 7.

De préférence, le tampon utilisé sera un tampon TRIS-HCl (solution de TRIS-HCl dans l'eau) ou un tampon Citrate-Phosphate (solution de Na₂HPO₄ et d'acide citrique dans l'eau), permettant en particulier d'atteindre un pH dans la gamme d'environ 6,5 à environ 7,5, plus particulièrement un pH d'environ 7.

Dans un mode de réalisation, la composition gélifiée, avantageusement stérile, selon l'invention peut contenir un composé supplémentaire comme un antibiotique, un substrat colorimétrique d'enzymes intervenant dans la résistance aux antibiotiques, un autre réactif colorimétrique spécifique de microorganismes, un substrat colorimétrique de toxines ayant une activité enzymatique ou une combinaison de ceux-ci.

Dans un mode de réalisation préféré, la composition gélifiée, avantageusement stérile, selon l'invention peut contenir un antibiotique. On peut citer à titre d'exemple comme antibiotique et sans vouloir se limiter l'ampicilline, kanamycine, tétracycline, chloramphénicol, carbénicilline, streptomycine, gentamicine, amoxicilline, sulfaméthoxazole, sulfachloropyridazine et une combinaison de ceux-ci.

Dans un mode de réalisation préféré, la composition gélifiée, avantageusement stérile, selon l'invention peut contenir un substrat colorimétrique d'enzymes intervenant dans la résistance aux antibiotiques. On peut citer à titre d'exemple comme substrat et sans vouloir se limiter la nitrocéfine, la carbapénème et une combinaison de ceux-ci.

Dans un mode de réalisation préféré, la composition gélifiée, avantageusement stérile, selon l'invention peut contenir un autre réactif colorimétrique spécifique de microorganismes. On peut citer à titre d'exemple comme substrat colorimétrique et sans vouloir se limiter le violet de gentiane, l'ortho-nitrophényl-β-galactopyranoside et le 4-méthylumbélliféryl-β-D-glucuronide.

Dans un mode de réalisation préféré, la composition gélifiée, avantageusement stérile, selon l'invention peut contenir un substrat colorimétrique de toxines ayant une activité enzymatique. On peut citer à titre d'exemple comme réactif colorimétrique et sans vouloir se limiter l'indophénol pour la détection d'activité AMP déaminase, les nanoparticules d'or pour la détection d'activité ribonucléase, le DNA-méthyl green pour la détection d'activité DNase et une combinaison de ceux-ci.

L'invention a également pour objet un procédé de détection de microorganismes viables sur une surface caractérisé en ce qu'il comprend au moins une fois l'ensemble des étapes successives suivantes :
- collecte d'un échantillon sur la surface à tester,
- mise en contact de l'échantillon avec une composition gélifiée, avantageusement stérile, selon l'invention,
- incubation de la composition gélifiée en contact avec l'échantillon au moins 3 heures, et
- observation de la couleur de la composition gélifiée.

La collecte de l'échantillon peut être effectuée par tout système de récupération de matière présente sur une surface tel qu'une éponge, une lingette, un filtre, une mousse, une gélose ou un écouvillon. De préférence, il s'agira d'un écouvillon qui peut être par exemple en coton, en rayonne, en fibre de polyester ou en nylon floqué.

La mise en contact de l'échantillon avec la composition gélifiée peut être effectuée par simple mise en contact du système de récupération de matière, après collecte de l'échantillon par frottement sur la surface, avec la composition gélifiée.

L'observation de la couleur pourra être effectuée avantageusement à l'œil nu. Si un changement de couleur ou l'apparition d'une couleur est observée (par rapport à la couleur (ou absence de couleur dans le cas du TTC ou de l'INT) initiale), cela signifie que des microorganismes viables sont présents sur la surface testée. Ce changement ou apparition de couleur se situera plus particulièrement au point de contact de l'échantillon avec la composition gélifiée.

De préférence, le procédé de détection de microorganismes viables sur une surface est caractérisé en ce qu'il comprend au moins une fois l'ensemble des étapes successives suivantes :
- collecte d'un échantillon avec un écouvillon sur la surface à tester,
- mise en contact dans un tube de l'écouvillon avec une composition gélifiée, avantageusement stérile, selon l'invention,
- incubation de la composition gélifiée en contact avec l'échantillon au moins 3 heures, et
- observation de la couleur de la composition gélifiée.

Selon l'invention, l'écouvillon peut être tout écouvillon en coton, rayonne, fibre de polyester ou nylon floqué.

L'invention a également pour objet un procédé de détection de microorganismes viables dans un fluide caractérisé en ce qu'il comprend au moins une fois l'ensemble des étapes successives suivantes :
- passage du fluide à tester dans un filtre,
- récupération du filtre et mise en contact dudit filtre avec une composition gélifiée, avantageusement stérile, selon l'invention,
- incubation de la composition gélifiée en contact avec le filtre au moins 3 heures, et
- observation de la couleur de la composition gélifiée.

Selon l'invention, on entend par filtre toute membrane, papier, tissu destiné à filtrer un fluide. Ce filtre peut être mais n'est pas limité à un filtre en nylon, cellulose, polyéthersulfone, poly(fluorure de vinylidène), ou polytétrafluoroéthylène.

L'observation de la couleur pourra être effectuée comme indiqué précédemment dans le cadre d'un procédé de détection de microorganismes viables sur une surface. Si un changement de couleur ou l'apparition d'une couleur est observée, cela signifie que des microorganismes viables sont présents dans le fluide. Ce changement ou apparition de couleur se situera plus particulièrement au point de contact du filtre avec la composition gélifiée.

Selon l'invention, les microorganismes détectés par ces procédés (sur une surface ou dans un fluide) peuvent être mais ne sont pas limités à des souches de *Bacillus subtilis, Escherichia coli, Staphylococcus aureus, Pseudomonas aeruginosa, Candida albicans, Saccharomyces cerevisiae, Streptococcus pyogenes, Streptococcus agalactiae, Cryptococcus macerans, Gluconobacter oxydans, Mycobacterium, Salmonella* ou une combinaison de celles-ci.

L'invention a également pour objet l'utilisation de la gomme gellane comme gélifiant d'une solution contenant au moins un sel de tétrazolium.

De préférence, la gomme gellane est utilisée comme gélifiant dans une composition selon l'invention dans laquelle le ou les sels de tétrazolium sont le MTT, le TTC, l'INT, le MTS, le WST-1, le WST-8, le XTT ou une combinaison de ceux-ci, de préférence le MTT, le TTC, l'INT, le WST-8 ou une combinaison de ceux-ci.

L'invention a également pour objet l'utilisation d'une composition gélifiée, avantageusement stérile, selon l'invention dans un kit de détection de viabilité cellulaire.

Selon l'invention, on entend par kit de détection tout dispositif de prélèvement d'échantillon pouvant mettre en contact l'échantillon et la composition gélifiée, avantageusement stérile, selon l'invention dans le but de détecter la présence ou l'absence de microorganismes viables dans l'échantillon.

Le kit de détection pourra ainsi comprendre :
- un récipient, de préférence un tube, avantageusement stérile, contenant la composition gélifiée, avantageusement stérile, selon l'invention, et
- un dispositif, avantageusement stérile, de prélèvement d'échantillon, plus particulièrement sur une surface ou dans un fluide.

Le dispositif de prélèvement d'échantillon sur une surface pourra être tout système de récupération de matière présente sur une surface tel qu'une éponge, une lingette, un filtre, une mousse, une gélose ou un écouvillon. De préférence, il s'agira d'un écouvillon qui peut être par exemple en coton, en rayonne, en fibre de polyester ou en nylon floqué.

Le dispositif de prélèvement d'échantillon dans un fluide sera en particulier un filtre qui peut être toute membrane, papier, tissu destiné à filtrer un fluide. Ce filtre peut être mais n'est pas limité à un filtre en nylon, cellulose, polyéthersulfone, poly(fluorure de vinylidène), ou polytétrafluoroéthylène.

La présente invention a également pour objet un tel kit de détection.

L'invention a également pour objet l'utilisation d'une composition gélifiée, avantageusement stérile, selon l'invention en tant que contrôle de contamination.

L'invention a également pour objet l'utilisation d'une composition gélifiée, avantageusement stérile, selon l'invention pour la détection de la viabilité cellulaire.

Dans un mode de réalisation, l'invention a pour objet l'utilisation d'une composition gélifiée, avantageusement stérile, pour la détection de la viabilité cellulaire et le contrôle de résistance à un ou plusieurs antibiotiques lorsque la composition gélifiée selon l'invention comprend en outre un ou plusieurs antibiotiques et un ou plusieurs substrats colorimétriques d'enzymes intervenant dans la résistance audit ou auxdits antibiotiques.

Dans un mode de réalisation, l'invention a pour objet l'utilisation d'une composition gélifiée, avantageusement stérile, pour la détection de la viabilité cellulaire et de toxines lorsque la composition gélifiée comprend en outre au moins un substrat colorimétrique de toxines ayant une activité enzymatique.

Dans un mode de réalisation, l'invention a pour objet l'utilisation d'une composition gélifiée, avantageusement stérile, pour la détection de la viabilité cellulaire et de la présence de microorganismes spécifiques lorsque la composition gélifiée comprend en outre au moins un autre réactif colorimétrique spécifique de microorganismes.

D'autres aspects, objets, avantages et caractéristiques de l'invention, seront présentés à la lecture de la description non restrictive qui suit et qui décrit différents tests et des modes de réalisations préférés de l'invention donnés par le biais d'exemples.

### EXEMPLES

### EXEMPLE 1 : Préparation des compositions et solutions de base utilisées dans les essais de compositions gélifiées à base de sel de tétrazolium

Un grand nombre de composés gélifiants ont été testés afin de pouvoir passer d'un test liquide faisant intervenir un sel de tétrazolium à un test gélifié. Chaque composition permettant de tester les gélifications comprend un tampon (Tris-HCl ou Citrate-Phosphate), un sel de tétrazolium (MTT, TTC ou INT) ainsi qu'un gélifiant.

### 1. Préparation du tampon

Pour les différents tests, un tampon Tris-HCl ou un tampon Citrate-Phosphate est utilisé.

Le tampon Tris-HCl (tr) préparé est un tampon Tris-HCl 50mM pH7. Ce tampon est préparé en diluant au 1/20^{e} une solution mère de Tris-HCl 1M (Sigma-Aldrich, solution de chlorhydrate) avec de l'eau déminéralisée stérile.

Le tampon Citrate-Phosphate (cp) préparé est un tampon Citrate-Phosphate pH7. Cette préparation est réalisée selon le protocole suivant :
- dissoudre 14,195g de Na₂HPO₄ dans 500mL d'eau déminéralisée (solution B),
- dissoudre 1,92g d'acide citrique dans 100mL d'eau déminéralisée (solution A),
- ajouter à la solution B de la solution A jusqu'à l'obtention d'un pH 7, et
- filtrer sur filtre stérile à membrane de polyéthersulfone 0,22µm (Thermo Scientific, Unités de filtration stériles avec membrane PES) le mélange des solutions A et B pour obtenir le tampon Citrate-Phosphate.

### 2. Préparation de la solution mère de sel de tétrazolium

Pour les différents tests, une solution mère de MTT, de TTC ou d'INT sont utilisées.

0,0083g de MTT (Sigma-Aldrich référence M2128-1G) sont dissouts dans 10mL d'eau déminéralisée stérile. Cette solution est filtrée sur filtre stérile d'acétate de cellulose de 0,2µm et conservée à 4°C à l'abri de la lumière.

0,0067g de TTC (Sigma-Aldrich référence T8877-5G) sont dissouts dans 10mL d'eau déminéralisée stérile. Cette solution est filtrée sur filtre stérile d'acétate de cellulose de 0,2µm et conservée à 4°C à l'abri de la lumière.

0,0101g d'INT (Sigma-Aldrich référence I10406-1G) sont dissouts dans 10mL d'eau déminéralisée stérile. Cette solution est filtrée sur filtre stérile d'acétate de cellulose de 0,2µm et conservée à 4°C à l'abri de la lumière.

### 3. Préparation de la composition de gélifiant.

Pour préparer les gélifiants, trois protocoles différents sont utilisés et un seul sera sélectionné en fonction du gélifiant et de ses conditions d'utilisations :
- dispersion à froid
- dispersion à chaud
- ajout de composé spécifique

Les gélifiants pour lesquels le protocole de dispersion à froid a été utilisé sont la gélatine issue de la peau de porc, le KAPPA carraghénane, la gomme arabique, la méthylcellulose et l'hydroxyéthylcellulose.

La préparation des gélifiants par dispersion à froid est réalisée selon le protocole suivant :
- dans un bécher de 250mL à 20°C et sous agitation magnétique, verser 100mL de tampon Tris-HCl ou de tampon Citrate-Phosphate,
- verser le gélifiant en poudre dans le bécher contenant le tampon jusqu'à l'obtention d'un mélange homogène, et
- chauffer le bécher jusqu'à la température optimale de solubilisation en laissant l'agitation magnétique jusqu'à l'obtention d'un gel.

Les gélifiants pour lesquels le protocole de dispersion à chaud a été utilisé sont l'agar agar, la pectine de peau de citron, la gomme xanthane, la gomme gellane et la gomme adragante.

La préparation des gélifiants par dispersion à chaud est réalisée selon le protocole suivant :
- dans un bécher de 250mL à température optimale de solubilisation et sous agitation magnétique, verser 100mL de tampon Tris-HCl ou de tampon Citrate-Phosphate,
- verser le gélifiant en poudre dans le bécher contenant le tampon jusqu'à l'obtention d'un mélange homogène, et
- garder le bécher à la température optimale de solubilisation en laissant l'agitation magnétique jusqu'à l'obtention d'un gel.

Le gélifiant pour lequel le protocole de dispersion avec ajout de composé spécifique est utilisé est l'alginate de sodium.

La préparation du gélifiant avec ajout de composé spécifique est réalisée selon le protocole suivant :
- dans un bécher de 250mL à 20°C et sous agitation magnétique, verser 50mL de tampon Tris-HCl ou de tampon Citrate-Phosphate,
- ajouter dans le bécher contenant le tampon 50mL de lactate de calcium à 5g/L,
- verser le gélifiant en poudre dans le bécher jusqu'à l'obtention d'un mélange homogène, et
- chauffer le bécher jusqu'à la température optimale de solubilisation en laissant l'agitation magnétique jusqu'à l'obtention un gel.

Pour plus de simplicité les masses de gélifiants et températures optimales de solubilisation des différents gélifiants ainsi que leur protocole de dispersion sont répertoriées dans le Tableau 1. Le tampon tr correspond au tampon TRIS-HCl et le tampon cp correspond au tampon Citrate-Phosphate.

**TABLEAU 1 :**

| **Gélifiant** | **Concentration gélifiant (g/100mL)** | **Protocole de dispersion** | **Température optimale** |
|---|---|---|---|
| Gélatine issue de la peau de porc | 2% | Dispersion à froid | 55-65°C |
| KAPPA Carraghénane | 0,5% | Dispersion à froid | 60-70°C |
| Gomme Adragante | 2% | Dispersion à chaud | 60-70°C |
| Gomme Arabique | 2% | Dispersion à froid | 60-70°C |

| **Gélifiant** | **Concentration gélifiant (g/100mL)** | **Protocole de dispersion** | **Température optimale** |
|---|---|---|---|
| Methylcellulose | 3% | Dispersion à froid | 60-70°C |
| Hydroxyéthylcellulose | 3% | Dispersion à froid | 60-70°C |
| Pectine de peau de citron | 2% | Dispersion à chaud | 55-65°C |
| Gomme Xanthane | 2% | Dispersion à chaud | 60-70°C |
| Alginate de Sodium | 2% | Ajout de composé spécifique (lactacte de calcium) | 60-70°C |
| Agar agar | 2% | Dispersion à chaud | 60-70°C |
| Gomme gellane | 1,25% | Dispersion à chaud | 65-75°C (cp)* ou 55-65°C (tr)** |

| | | | |
|---|---|---|---|
| * température optimale dans le cas de l'utilisation du tampon Citrate-Phosphate (cp) ** température optimale dans le cas de l'utilisation du tampon TRIS-HCl (tr) | | | |

### EXEMPLE 2 : Essais de compositions gélifiées à base de sel de tétrazolium

### 1. Variation du gélifiant.

Le but de la gélification est d'obtenir un gel inerte par rapport au sel de tétrazolium. Plus précisément, le gélifiant ne doit pas activer spontanément la coloration du sel de tétrazolium et ne doit pas empêcher l'observation de la coloration tout en donnant un gel non friable ou cassant.

Pour tester les compositions gélifiées de sel de tétrazolium, un mélange avec des proportions de 5 volumes de mélange tampon (cp)/gélifiant pour 1 volume de solution mère de sel de tétrazolium est réalisé, les mélanges tampon (cp)/gélifiant et les solutions mères de sel de tétrazolium ayant été préparés selon les protocoles de l'exemple 1.

Afin d'être certain que le gélifiant n'active pas le ou les sels de tétrazolium utilisés, un suivi de la coloration des différentes compositions a été réalisé sur plusieurs heures. Au préalable, les compositions gélifiées sont passées à l'autoclave à 110°C pendant 45 minutes pour être certain de leur stérilité.

Le Tableau 2 présente les résultats des compositions gélifiées avec différents gélifiants et un tampon TRIS-HCl (tr). Chaque composition a été évaluée selon trois critères : si la composition s'est bien gélifiée avec obtention d'un gel non friable ou cassant (gélification faible, moyenne ou forte), si le sel de tétrazolium (MTT, INT ou TTC) s'est activé spontanément (activation du sel) et si la composition a une coloration ou une opacité empêchant de voir correctement le signal lié à la coloration du sel de tétrazolium (lisibilité du signal).

Les mêmes résultats ont été obtenus quel que soit le sel de tétrazolium testé (MTT, INT ou TTC).

**TABLEAU 2 :**

| **Gélifiant** | **Gélification** | **Activation du sel** | **Lisibilité du signal** |
|---|---|---|---|
| Gélatine issue de la peau de porc | Moyenne | Non | Signal lisible |
| KAPPA Carraghénane | Moyenne | Non | Signal lisible |
| Gomme Adragante | Moyenne | Non | Signal illisible |
| Gomme Arabique (cp) | Faible | Non | Signal illisible |
| Méthylcellulose | Faible | Non | Signal lisible |
| Hydroxyéthylcellulose | Faible | Oui | Signal illisible |
| Pectine de peau de citron | Faible | Oui | Signal illisible |
| Gomme Xanthane | Forte | Non | Signal illisible |
| Alginate de Sodium | Faible | Non | Signal lisible |
| Agar agar | Forte | Oui | Signal illisible |
| Gomme gellane | Forte | Non | Signal lisible |

Les résultats du Tableau 2 montrent que la gélatine de peau de porc, le KAPPA carraghénane, la gomme adragante, la gomme arabique, la méthylcellulose, l'hydroxyéthylcellulose, la pectine de peau de citron et l'alginate de sodium ne permettent pas d'obtenir une gélification correcte de la composition. Ces composés ne sont donc pas utilisables en tant que gélifiants de la composition selon l'invention. Seul l'agar agar, la gomme xanthane et la gomme gellane permettent une bonne gélification de la composition.

Néanmoins, l'agar agar active spontanément la coloration du sel de tétrazolium donc l'agar agar n'est pas non plus utilisable en tant que gélifiant.

La gomme xanthane ne permet pas une bonne lecture du signal donc la gomme xanthane n'est pas non plus utilisable en tant que gélifiant.

De tous les composés testés seule la gomme gellane permet d'avoir une composition gélifiée ayant de bonnes propriétés de gélification, n'activant pas spontanément la coloration due au sel de tétrazolium et dont le signal reste visible tout en donnant un gel compact.

### 2. Variation du tampon.

Des compositions gélifiées de sel de tétrazolium utilisant la gomme de gellane comme gélifiant ont également été réalisées avec différents systèmes tampon. Pour cela un mélange avec des proportions de 5 volumes de mélange tampon/ gomme gellane pour 1 volume de solution mère de sel de tétrazolium est réalisé, le mélange tampon/gomme gellane et les solutions mères de sel de tétrazolium ayant été préparés selon les protocoles de l'exemple 1.

Le tableau 3 présente les résultats obtenus.

**TABLEAU 3 :**

| **Gélifiant** | **Tampon** | **Gélification** | **Activation du sel** | **Lisibilité du signal** |
|---|---|---|---|---|
| Gomme gellane | tr | Forte | Non | Signal lisible |
| Gomme gellane | cp | Forte | Non | Signal lisible |

Divers tampons (tr ou cp) peuvent ainsi être utilisés.

### EXEMPLE 3 : Préparation d'une composition comprenant au moins deux sels de tétrazolium

Pour les différentes préparations, des solutions mères de MTT, de TTC et/ou d'INT sont utilisées et seront mélangées entre elles en fonction de la composition de sels de tétrazolium désirée.

Pour une composition comprenant deux sels de tétrazolium, le protocole de préparation est le suivant :
- préparer un tampon de Tris-HCl ou de Citrate-Phosphate selon le protocole de l'exemple 1,
- préparer le gélifiant à partir de gomme gellane selon le protocole de l'exemple 3,
- dissoudre chaque sel de tétrazolium dans 5mL d'eau déminéralisée stérile,
- filtrer chaque solution de sel de tétrazolium sur filtre stérile d'acétate de cellulose de 0,2µm et conserver à 4°C à l'abri de la lumière,
- mélanger les solutions de sels désirées pour avoir un volume final de 10mL, et
- mélanger et homogénéiser 5mL de la solution contenant les deux sels de tétrazolium à 20mL de solution contenant le mélange tampon/gélifiant.

Pour une composition comprenant les trois sels de tétrazolium, le protocole de préparation est le suivant :
- préparer un tampon de Tris-HCl ou de Citrate-Phosphate selon le protocole de l'exemple 1,
- préparer le gélifiant à partir de gomme gellane selon le protocole présenté dans l'exemple 3,
- dissoudre chaque sel de tétrazolium dans 5mL d'eau déminéralisé stérile,
- filtrer chaque solution de sel de tétrazolium sur filtre stérile d'acétate de cellulose de 0,2µm et conserver à 4°C à l'abri de la lumière,
- mélanger et homogénéiser les solutions de sels désirées pour avoir un volume final de 15mL, et
- mélanger 5mL de la solution contenant les trois sels de tétrazolium à 20mL de solution contenant le mélange tampon/gélifiant.

Les compositions des solutions contenant deux ou trois sels de tétrazolium différents, la masse de sels de tétrazolium à peser et les volumes finaux des solutions sont présentés dans le Tableau 4.

**TABLEAU 4 : Résumé des compositions contenant deux ou trois sels de tétrazolium.**

| **Compositions** | **Masse de MTT** | **Masse d'INT** | **Masse de TTC** | **Volume final de la composition** |
|---|---|---|---|---|
| MTT/INT | 0,0041g | 0,0050g | / | 10mL |
| MTT/TTC | 0,0041g | / | 0,0034g | 10mL |
| INT/TTC | / | 0,0050g | 0,0034g | 10mL |
| MTT/TTC/INT | 0,0014g | 0,0034g | 0,0022g | 10mL |
| MTT/TTC/INT | 0,0041g | 0,0050g | 0,0034g | 15mL |

### EXEMPLE 4 : Préparation d'une composition gélifiée pour un kit de détection à base de MTT, TTC ou INT

### 1. Préparation du gélifiant.

0,3125g de gomme gellane (Sigma-Aldrich référence P8169-100G) sont pesés et mélangés à 20mL de tampon Tris-HCl ou de tampon Citrate-Phosphate. Le mélange est ensuite autoclavé 45 minutes à 110°C pour la dissolution de la gomme gellane et sa stérilisation.

### 2. Préparation des tubes de test.

- ajouter 5mL de la solution mère de sel de tétrazolium selon le protocole décrit à l'exemple 1 au mélange tampon/gélifiant sorti d'autoclave, afin d'obtenir un volume final de 25mL,
- homogénéiser le mélange et aliquoter en tubes stériles de 5mL, et
- laisser refroidir pour que le mélange se solidifie.

### EXEMPLE 5 : Préparation d'une composition gélifiée pour un kit de détection à base de WST-8.

### 1. Préparation du gélifiant.

0.3g de gomme gellane (Sigma-Aldrich référence P8169-100G) sont pesés et mélangés à 15mL de tampon Tris-HCl. Le mélange est ensuite autoclavé 45 minutes à 110°C pour la dissolution de la gomme gellane et sa stérilisation.

### 2. Préparation des tubes de test.

Préparation d'une solution WST-8 : 0.015g de WST-8 (Interchim) sont dissouts dans 5mL de Tampon Tris-HCl 50mM pH7. Cette solution est filtrée sur filtre stérile d'acétate de cellulose de 0,2µm et conservée à 4°C à l'abri de la lumière.

Préparation d'une solution de mPMS : 0.00067g de mPMS (1-méthoxy-5-méthylphénazinium méthyl sulfate - SIGMA) sont dissouts dans 2mL de Tris-HCl 50mM pH7.

### Préparation des tubes tests :

- ajouter à 12.5mL de gélifiant préparé selon le protocole du point 1 :
   - 5mL de solution WST-8,
   - 1.5mL de solution de mPMS,
   - 6mL de tampon Tris-HCl 50mM pH7, afin d'obtenir un volume final de 25mL,
- homogénéiser le mélange et aliquoter en tubes stériles par fractions de 2.5mL, et
- laisser refroidir pour que le mélange se solidifie.

### EXEMPLE 6 : Utilisation d'une composition gélifiée à base de WST-8 pour un la détection de microorganismes viables.

### 1. Préparation des tubes de test.

Une solution de gomme gellane à 2% a été autoclavée 20 mins à 121°C. Une solution à 120µM de mPMS et 1mM de WST-8 a été préparée et stérilisée par filtration sur filtre 0.22µm. Après refroidissement de la solution de gomme gellane à environ 60°C, les deux solutions ont été mélangées volume à volume afin d'obtenir des concentrations en mPMS, WST-8 et gellane respectivement de 60µM, 0.5mM et 1%. Le mélange a été aliquoté par 1mL en tube stérile de 10mL et laissé à température ambiante jusqu'à gélification.

### 2. Préparation des échantillons de microorganismes.

Trois souches ont été choisies, afin d'évaluer les performances et les seuils de détection des compositions : *Bacillus subtilis, Pseudomonas aeruginosa* et *Escherichia coli.*

Pour chaque souche, une gamme de dix échantillons a été préparée, chaque échantillon contenant un nombre de cellules de bactérie s'échelonnant de 0, 1, 10¹, 10², 10³, 10⁴, 10⁵, 10⁶, 10⁷ et 10⁸, dans un volume de 40µL de milieu de culture adapté. Le milieu LB est utilisé pour *E. coli* et *B. subtilis.* Pour *P. aeruginosa,* un milieu a été préparé comme suit : 15g/L de pepticase, 5g/L de Bacto Soytone et 5g/L de NaCl à pH 7.3.

### 3. Essais de détection de microorganismes viables

Chaque échantillon a été absorbé sur un écouvillon, puis l'écouvillon a été introduit dans la composition gélifiée d'un tube de test. Les tubes ont ensuite été incubés dans une étuve à 37°C. L'apparition de la couleur orange a été suivie pendant 24h à l'aide d'une caméra.

L'observation des tubes a permis de déterminer le temps nécessaire à l'apparition d'un signal positif pour chacune des trois souches. Le tableau 5 ci-dessous présente les temps nécessaires pour détecter 10¹, 10³ et 10⁶ cellules.

**TABLEAU 5 : Temps de détection en fonction du nombre de cellules**

| **Souche** | **Nombre de cellules** | | |
|---|---|---|---|
| | **10⁶** | **10³** | **10¹** |
| *B. subtilis* | 4h | 7-8h | 9-10h |
| *P. aeruginosa* | 4h | 9-10h | 11-12h |
| *E. coli* | 6h | 14-15h | 19-20h |

## Revendications

1. Composition gélifiée stérile comprenant au moins un sel de tétrazolium, un tampon et de la gomme gellane.

2. Composition gélifiée selon la revendication 1 dans laquelle le tampon est un tampon citrate-phosphate ou un tampon Tris-HCl.

3. Composition gélifiée selon la revendication 1 ou 2 dans laquelle le ou les sels de tétrazolium sont du MTT, de l'INT, du TTC, du MTS, du WST-1, du WST-8, du XTT ou un mélange de ceux-ci ; de préférence du MTT, de l'INT, du TTC, du WST-8 ou un mélange de ceux-ci.

4. Composition gélifiée selon l'une quelconque des revendications 1 à 3 comprenant en outre un antibiotique, un substrat colorimétrique d'enzyme de résistance à un antibiotique, un autre réactif colorimétrique spécifique de microorganismes, un substrat colorimétrique de toxines ayant une activité enzymatique ou une combinaison de ceux-ci.

5. Procédé de détection de microorganismes viables sur une surface comprenant au moins une fois l'ensemble des étapes successives suivantes :
- collecte d'un échantillon sur la surface à tester,
- mise en contact de l'échantillon avec une composition gélifiée selon l'une quelconque des revendications 1 à 4,
- incubation de la composition gélifiée en contact avec l'échantillon au moins 3 heures, et
- observation de la couleur de la composition gélifiée.

6. Procédé de détection selon la revendication 5, dans lequel :
- la collecte de l'échantillon sur la surface à tester est réalisée avec un écouvillon, et
- la mise en contact de l'échantillon avec la composition gélifiée selon l'une quelconque des revendications 1 à 4 est réalisée dans un tube.

7. Procédé de détection de microorganismes viables dans un fluide comprenant au moins une fois l'ensemble des étapes successives suivantes :
- passage du fluide à tester dans un filtre,
- récupération du filtre et mise en contact dudit filtre avec une composition gélifiée selon l'une quelconque des revendications 1 à 4,
- incubation de la composition gélifiée en contact avec le filtre au moins 3 heures, et
- observation de la couleur de la composition gélifiée.

8. Procédé de détection selon l'une quelconque des revendications 5 à 7 dans lequel les microorganismes sont des souches de *Bacillus subtilis, Escherichia coli, Staphylococcus aureus, Pseudomonas aeruginosa, Candida albicans, Saccharomyces cerevisiae, Streptococcus pyogenes, Streptococcus agalactiae, Cryptococcus macerans, Gluconobacter oxydans, Mycobacterium, Salmonella* ou une combinaison de celles-ci.

9. Utilisation d'une composition gélifiée selon l'une quelconque des revendications 1 à 4 pour la détection de viabilité cellulaire.

10. Utilisation d'une composition gélifiée selon l'une quelconque des revendications 1 à 4 en tant que contrôle de contamination par des microorganismes.

11. Utilisation d'une composition gélifiée selon la revendication 4 comprenant un ou plusieurs antibiotiques et un ou plusieurs substrats colorimétriques d'enzymes de résistance audit ou auxdits antibiotiques, pour la détection de la viabilité cellulaire et le contrôle de résistance à un ou plusieurs antibiotiques.

12. Utilisation d'une composition gélifiée selon la revendication 4 comprenant un substrat colorimétrique de toxines ayant une activité enzymatique, pour la détection de la viabilité cellulaire et la détection de toxines.

13. Utilisation d'une composition gélifiée selon la revendication 4 comprenant un autre réactif colorimétrique spécifique de microorganismes, pour la détection de la viabilité cellulaire et de la présence de microorganismes spécifiques.

14. Utilisation d'une composition gélifiée selon l'une quelconque des revendications 1 à 4 dans un kit de détection de viabilité cellulaire.

15. Kit de détection de viabilité cellulaire comprenant :
- un récipient contenant une composition gélifiée selon l'une quelconque des revendications 1 à 4, et
- un dispositif de prélèvement d'échantillon.

## Patentansprüche

1. Gelartige sterile Zusammensetzung, umfassend wenigstens ein Tetrazoliumsalz, einen Puffer und Gellangummi.

2. Gelartige Zusammensetzung nach Anspruch 1, in welcher der Puffer ein Citrat-Phosphat-Puffer oder ein Tris-HCl-Puffer ist.

3. Gelartige Zusammensetzung nach Anspruch 1 oder 2, in welcher das oder die Tetrazoliumsalze MTT, INT, TTC, MTS, WST-1, WST-8, XTT oder eine Mischung davon sind; bevorzugt MTT, INT, TTC, WST-8 oder eine Mischung davon.

4. Gelartige Zusammensetzung nach einem der Ansprüche 1 bis 3, weiter umfassend ein Antibiotikum, ein kolorimetrisches Substrat eines Resistenzenzyms gegen ein Antibiotikum, ein weiteres kolorimetrisches, spezifisches Mikroorganismen-Reagens, ein kolorimetrisches Toxin-Substrat mit einer enzymatischen Aktivität oder eine Kombination davon.

5. Detektionsverfahren von viablen Mikroorganismen auf einer Oberfläche, wenigstens einmal die folgenden aufeinanderfolgenden Schritte umfassend:
- Entnahme einer Probe auf der zu testenden Oberfläche,
- Inkontaktbringen der Probe mit einer gelartigen Zusammensetzung nach einem der Ansprüche 1 bis 4,
- Inkubation der gelartigen Zusammensetzung in Kontakt mit der Probe für mindestens 3 Stunden, und
- Observieren der Farbe der gelartigen Zusammensetzung.

6. Detektionsverfahren nach Anspruch 5, in welchem:
- die Entnahme der Probe auf der zu testenden Oberfläche mit einem Abstrichtupfer durchgeführt wird, und
- das Inkontaktbringen der Probe mit der gelartigen Zusammensetzung nach einem der Ansprüche 1 bis 4 in einer Röhre durchgeführt wird.

7. Detektionsverfahren für viable Mikroorganismen in einem Fluid, wenigstens einmal die Gesamtheit der folgenden aufeinanderfolgenden Schritte umfassend:
- Durchlassen des zu testenden Fluids durch einen Filter,
- Rückgewinnung des Filters und Inkontaktbringen des Filters mit einer gelartigen Zusammensetzung nach einem der Ansprüche 1 bis 4,
- Inkubation der gelartigen Zusammensetzung in Kontakt mit dem Filter für wenigstens 3 Stunden, und
- Observieren der Farbe der gelartigen Zusammensetzung.

8. Detektionsverfahren nach einem der Ansprüche 5 bis 7, in welchem die Mikroorganismen von *Bacillus subtilis, Escherichia coli, Staphylococcus aureus, Pseudomonas aeruginosa, Candida albicans, Saccharomyces cerevisiae, Streptococcus pyogenes, Streptococcus agalactiae, Cryptococcus macerans, Gluconobacter oxydans, Mycobacterium, Salmonellen* oder einer Kombination davon abstammen.

9. Verwendung einer gelartigen Zusammensetzung nach einem der Ansprüche 1 bis 4 für die Detektion von zellulärer Viabilität.

10. Verwendung einer gelartigen Zusammensetzung nach einem der Ansprüche 1 bis 4 als Kontrolle für Kontaminierung durch Mikroorganismen.

11. Verwendung einer gelartigen Zusammensetzung nach Anspruch 4, umfassend ein oder mehrere Antibiotika und ein oder mehrere kolorimetrische Substrate von Resistenzenzymen gegen das oder die Antibiotika für die Detektion der zellulären Viabilität und die Kontrolle der Resistenz gegen ein oder mehrere Antibiotika.

12. Verwendung einer gelartigen Zusammensetzung nach Anspruch 4, umfassend ein kolorimetrisches Toxin-Substrat mit einer enzymatischen Aktivität, für die Detektion der zellulären Viabilität und die Toxindetektion.

13. Verwendung einer gelartigen Zusammensetzung nach Anspruch 4, umfassend ein weiteres kolorimetrisches spezifisches Mikroorganismen-Reagens für die Detektion der zellulären Viabilität und der Präsenz von spezifischen Mikroorganismen.

14. Verwendung einer gelartigen Zusammensetzung nach einem der Ansprüche 1 bis 4 in einem Detektionskit für zelluläre Viabilität.

15. Detektionskit für zelluläre Viabilität, Folgendes umfassend:
- ein Gefäß, umfassend eine gelartige Zusammensetzung nach einem der Ansprüche 1 bis 4, und
- eine Vorrichtung zur Probenentnahme.

## Claims

1. Sterile gelled composition comprising at least a tetrazolium salt, a buffer and gellan gum.

2. Gelled composition according to claim 1 wherein the buffer is a citrate-phosphate buffer or a Tris-HCl buffer.

3. Gelled composition according to claim 1 or 2 wherein the tetrazolium salt(s) are MTT, INT, TTC, MTS, WST-1, WST-8, XTT or a mixture thereof, preferably MTT, INT, TTC, WST-8 or a mixture thereof.

4. Gelled composition according to any one of claims 1 to 3 further comprising an antibiotic, a colorimetric substrate of an enzyme of resistance to an antibiotic, another colorimetric reagent specific to microorganisms, a colorimetric substrate of toxins having an enzymatic activity or a combination thereof.

5. Method for detecting viable microorganisms on a surface comprising at least once all of the following successive steps:
- collecting a sample on the surface to be tested,
- contacting the sample with a gelled composition according to any one of claims 1 to 4,
- incubating the gelled composition in contact with the sample at least 3 hours, and
- observing the colour of the gelled composition.

6. Detection method according to claim 5, wherein:
- collecting the sample on the surface to be tested is carried out with a swab, and
- contacting the sample with the gelled composition according to any one of claims 1 to 4 is carried out in a tube.

7. Method for detecting viable microorganisms in a fluid comprising at least once all of the following successive steps:
- passing the fluid to be tested through a filter,
- recovering the filter and contacting said filter with a gelled composition according to any one of claims 1 to 4,
- incubating the gelled composition in contact with the filter at least 3 hours, and
- observing the colour of the gelled composition.

8. Detection method according to any one of claims 5 to 7 wherein the microorganisms are strains of *Bacillus subtilis, Escherichia coli, Staphylococcus aureus, Pseudomonas aeruginosa, Candida albicans, Saccharomyces cerevisiae, Streptococcus pyogenes, Streptococcus agalactiae, Cryptococcus macerans, Gluconobacter oxydans, Mycobacterium, Salmonella* or a combination thereof.

9. Use of a gelled composition according to any one of claims 1 to 4 for the detection of cell viability.

10. Use of a gelled composition according to any one of claims 1 to 4 as a check for contamination by microorganisms.

11. Use of a gelled composition according to claim 4 comprising one or more antibiotics and one or more colorimetric substrates of enzymes of resistance to said antibiotic(s), for the detection of cell viability and checking for resistance to one or more antibiotics.

12. Use of a gelled composition according to claim 4 comprising a colorimetric substrate of toxins having an enzymatic activity, for the detection of cell viability and the detection of toxins.

13. Use of a gelled composition according to claim 4 comprising another colorimetric reagent specific to microorganisms, for the detection of cell viability and the presence of specific microorganisms.

14. Use of a gelled composition according to any one of claims 1 to 4 in a kit for detecting cell viability.

15. Kit for detecting cell viability comprising:
- a container containing a gelled composition according to any one of claims 1 to 4, and
- a device for sample collection.
